# EUROPEAN PATENT SPECIFICATION

(11) **EP 1 570 752 B1**
(45) Date of publication and mention of the grant of the patent: **02.07.2008**
(21) Application number: 04005383.7
(22) Date of filing: 06.03.2004
(51) Int. Cl.: A23L 1/30, A61K 31/201

(54) **Verwendung von ungesättigten Fettsäuren zur Verringerung des Appetits oder der Essensaufnahme**
Use of unsaturated fatty acids for the reduction of appetite or food intake
Utilisation des acides gras insaturés pour la diminution de l'appétit ou de l'apport alimentaire

(43) Date of publication of application: 07.09.2005
(73) Proprietor: Cognis IP Management GmbH, 40589 Düsseldorf (DE)
(72) Inventor: Bell, Doris, DE-40627 Düsseldorf (DE); Zink, Ralf, DE-40764 Langenfeld (DE); Weiss, Albrecht, DE-40764 Langenfeld (DE)

(56) References cited:
- US-A1- 2002 082 436
- KAMPHUIS M M J W ET AL: "Effect of conjugated linoleic acid supplementation after weight loss on appetite and food intake in overweight subjects." EUROPEAN JOURNAL OF CLINICAL NUTRITION. OCT 2003, vol. 57, no. 10, October 2003 (2003-10), pages 1268-1274, XP008032316 ISSN: 0954-3007
- MEDINA EDWARD A ET AL: "Conjugated linoleic acid supplementation in humans: Effects on circulating leptin concentrations and appetite" LIPIDS, vol. 35, no. 7, July 2000 (2000-07), pages 783-788, XP008032315 ISSN: 0024-4201
- MOYA-CAMARENA SILVIA Y ET AL: "Conjugated linoleic acid is a potent naturally occurring ligand and activator of PPARalpha" JOURNAL OF LIPID RESEARCH, vol. 40, no. 8, August 1999 (1999-08), pages 1426-1433, XP001196994 ISSN: 0022-2275

## Description

### Object of the invention

The present invention generally relates to the area of nutrition and pharmacy and in particular it relates to a method to decrease appetite and food intake.

### State of the art

Obesity is a serious health problem due to follow-up diseases such as for example cardiovascular problems, diabetes or arteriosclerosis.
Conjugated fatty acids such as conjugated linoleic acid (CLA) and its derivatives are known to have an influence on body fat, lean muscle mass and body weight. The state of the art shows published data and investigations which seem to be partly contradictory.
The European patent EP 0579901 B1 discloses the use of conjugated linoleic acid, free linoleic acid, salts thereof and mixtures thereof for the manufacture of a composition for preventing weight loss, reduction in weight gain or anorexia in an animal or human caused by immune stimulation. It shows that animal feed or human food which contains added conjugated linoleic acids can enhance growth and prevent anorexia and weight loss. The proposed mechanism is the modulation of the immune stimulation and the adverse effects of catabolic hormones. A similar indication is claimed in EP 0680318 B1 describing a method of enhancing weight gain and feed efficiency in an animal which comprises administering to the animal a safe and effective amount of a conjugated linoleic acid. Examples clearly show that CLA has a substantial positive effect on weight gain in animals.

In US5554646 A the effects of the administration of CLA are differentiated. Disclosed is a method of reducing body fat which comprises administering to the animal a safe and effective amount of a conjugated linoleic acid. Methods of preserving or increasing the animal's body protein by administering the conjugated linoleic acid also are disclosed.

A method of treating obesity and reducing body weight comprising the oral administration of conjugated linoleic acid was claimed in the US-patent US 6034132. It is proposed that this method is a safe intervention of the natural biochemical and physiological processes which direct food into tissue mass - opposite to chemical remedies which result in weight loss through reduced food intake and appetite suppression.

European Journal of Clinical Nutrition [(2003) 57, p. 1268-1274] discloses that Tonalin™, which is a mixture of CLA, has an appetite reducing effect and that CLA affects body weight.

The US patent application US 2003/0018081 A1 discloses methods, compounds, and compositions for reducing body fat and modulating fatty acid metabolism. It describes that fatty acidalkanolamides, especially oleylethanolamide, reduce appetite and food intake and can be used in the treatment of obesity. The ethanolamide moiety seems to be crucial for the binding of receptor binding of the molecule.
Recently it was investigated that oleylethanolamide regulates feeding and body weight through activation of the nuclear receptor PPAR-alpha (peroxisome-proliferator-activated receptor -alpha) [Fu et al.; Nature, Vol. 425; 4 September 2003; p.90 to 93]. This receptor type could also be activated by conjugated linoleic acid [Moya-Camarena S.Y., Vanden Heuvel J.P., Blanchard S.G. Leesnitzer L.A. & Belury M.A. Journal of Lipid Research 40; 1426-1433; 1999].PPAR receptors modulate the expression of various genes, which interfere with the metabolism and transport of lipids, with glucosehomeostasis, cell proliferation and differentiation and with apoptosis. Various subtypes such as PPAR alpha, beta (= delta) und gamma are expressed in different tissues.

It has been an object of the present invention to find new safe and healthy ways for the treatment of obesity, useful to prevent and fight weight gain without influencing the hormonal route.

### Detailed description of the invention

Object of the invention is the use of a substance which activates the nuclear receptor PPAR-alpha for the manufacture of a dietary supplement, a functional food, or a medicament for reducing appetite or food intake, preferably the use of conjugated fatty acids for the manufacture of a medicament for reducing appetite or food intake.
It has been found that the oral application of unsaturated fatty acids, especially conjugated fatty acids, especially conjugated linoleic acid (CLA) results in a reduction of appetite and in an inducement of satiety so that consequently food intake is decreased. Conjugated fatty acids, preferably CLA could therefore be used to prevent or treat obesity and manage weight loss via appetite reduction. Decreased intake of food due to administration of a natural occurring and essential food agent is a very safe and healthy way of reducing body weight.
We have discovered a method of inducing satiety in an animal or human which comprises administering a safe amount of CLA selected from 9,11-octadecadienoic acid salts of CLA such as the sodium or potassium salt of CLA.

Surprisingly it has been found that the *cis*-9,*trans*-11 isomer of conjugated linoleic acid - compared to other CLA isomers in general and, more specifically, compared to non-conjugated linoleic acid - shows this effect more pronounced, so that the CLA used for the manufacture of a medicament reducing appetite or food intake should have a remarkable amount of this isomer.
Therefore it is an object of the invention to use conjugated linoleic acid (CLA), comprising said *cis*-9,*trans*-11 isomer in amounts of at least 30 % b.w. ― calculated on the total CLA content for the manufacture of a medicament for reducing appetite or food intake and the use of a CLA composition with a high purity, the use of conjugated linoleic acid (CLA), comprising at most 30 % b.w. *trans*-10,*cis*-12 isomers and in total less than 1 % b.w. 8, 10-, 11, 13-*trans,trans* isomers ― calculated on the total CLA content for the manufacture of a medicament for reducing appetite or food intake.
CLA-compositions resulting from a commercially available process are usually ― but not necessarily - obtained by base-catalysed isomerisation of sunflower oil or their respective alkyl esters and subsequent isomerisation in the presence of enzymes. This process often results in an isomer distribution of the main isomers *cis-*9,*trans*-11 isomer and *trans*-10, *cis*-12 isomer in a ratio 1 : 1.2 to 1.2 : 1, which could directly be used for the manufacture of a medicament for reducing appetite or food intake. To achieve higher concentrations of the preferred c9 t11 isomer, selective enzymatic processes, or crystallization processes, or synthesis pathways different to the above mentioned are possible.

### Coniugated fatty acids

Conjugated fatty acids refers to acids having at least a hydrocarbon chain, with at least three consecutive carbon-carbon bonds, such that single and double carbon-carbon bonds are found in an alternating manner. These di- or poly-unsaturated acids are referred to herein using the common names of the corresponding naturally-occurring compounds having the same number of carbons and unsaturations. Although such naturally-occurring compounds are not necessarily conjugated, due to the arrangement of their carbon- carbon double bonds, it will be understood in the context of the present invention that only conjugated versions of those compounds are contemplated; i.e., the arrangement of the double bounds will be such that they contain the substructure C=C--C=C. While compounds having as few as 4, 5, 6, or 7 carbon atoms are contemplated, the preferred conjugated compounds have 8, 9, 10, 12, 14, 16 or more carbon atoms, preferably not more than 32, 30, 28, or 26 carbon atoms. This also includes all isomers of fatty acids.
Suitable conjugated fatty acids include, without limitation, conjugated versions of linoleic acid, linolenic acid, gamma linolenic acid, arachidonic acid, mead acid, stearidonic acid, alpha- eleostearic acid, eleostearic acid, pinolenic acid, docosatetraenoic acid, 9,12-octadecadienoic acid, octadecatrienoic acid, eicosatetraenoic acid, eicosapentaenoic acid, docosahexaenoic acid, docosapentaenoic acid, and all other diunsaturated and polyunsaturated fatty acids. In a preferred embodiment, the conjugated fatty acid is conjugated linoleic acid (CLA).

### Conjugated linoleic acid

As used herein, "conjugated linoleic acid" or "CLA" refers to any conjugated linoleic acid or octadecadienoic free fatty acid and is an acronym used for positional and geometric isomers deriving from the essential fatty acid linoleic acid (LA, cis-9,cis-12-octadecadienoic acid, 18:2n-6). It is intended that the term "CLA" includes all positional and geometric isomers of linoleic acid with two conjugated carbon-carbon double bonds any place in the molecule such as cis- and trans isomers ("E/Z isomers") of the following positional isomers: 2,4-octadecadienoic acid, 4,6-octadecadienoic acid, 6,8-octadecadienoic acid, 7,9-octadecadienoic acid, 8,10-octadecadienoic acid, 9,11-octadecadienoic acid and 10,12 octadecadienoic acid, 11, 13 octadecadienoic acid.
As used herein, "CLA" encompasses a single isomer and a selected mixture of two or more isomers.
CLA represents a commercially available product which usually is obtained by base-catalysed isomerisation of sunflower oil or their respective alkyl esters and subsequent isomerisation in the presence of enzymes. Usually a high percentage of linoleic acid is converted primarily to the conjugated c9,t11 and t10,c12 isomers in a carefully controlled reaction yielding greater than 90 percent of these isomers, so that less than a combined 1 percent of the 11,13 isomers, less than 1 percent of the 8,10 isomers, less than 1 percent of the double trans species (the t9,t11 and t10,t12 isomers), and less than 1 percent total unidentified linoleic acid species are present. A process as described in DE 10236086 results in a distribution of the main isomers *cis*-9,*trans*-11 isomer and *trans*-10, *cis*-12 isomer at equal parts, in a ratio of 1 : 1.2 to 1.2 : 1.

CLA has several structural and functional properties that are different from those of all-cis-nonconjugated polyunsaturated fatty acids. Emerging evidence has indicated that individual CLA isomers act differently in the biological systems and contribute differently in their beneficial or potential side effects. The *cis*-9,*trans*-11 CLA-isomer was most efficacious in inhibiting appetite. Until now, however, there is no specific way to predict the properties of certain isomers, therefore, the identification of new applications and uses is still a question of trial and error. From a physiological point of view the use of the *cis*-9,*trans*-11 isomer according to the present invention is of special importance having at least 30, preferably at least 50 and most preferably at least 80 % b.w. of said *cis*-9,*trans*-11 isomer ― calculated on the total CLA content of the crude mixture. In addition, it has been found advantageous if the content of the *trans*-10,*cis*-12 isomer is at most 45, preferably at most 10 % b.w. and the sum of 8,10-, 11,13- and *trans,trans-*isomers in total is less than 1 % b.w. ― again calculated on the total CLA content. Such products can be found in the market for example under the trademark To-nalin CLA-80 (Cognis). Usually, the daily dosage of CLA is 0.05 to 5, preferably 2.0 to 4 g/day. The compositions of CLA can contain the sodium or potassium salt of CLA in combination with a diluent.

In a preferred form of the invention, the CLA is offered as a free fatty acid, as a mono- or di-acylglyceride. It is important to make the substance easily available in its unesterified form, either in order to flood in the blood plasma to bind to fatty acid receptors presumably located in the lower hypothalamus (called Nucleus paraventricularis) which is not restricted by the blood-brain barrier; or to be directly transported to the liver through the portal vein, where the high offer of free fatty acids induces the synthesis of keton bodies which are known to induce satiety in the brain once released into the bloodstream. Other explanations and mechanisms of inducing satiety have for a longer time been missing, and have been subject of intensive investigation, e.g., the effect of the so-called gut hormones such as cholecystokinin. The herein described effect of unsaturated fatty acids binding to PPAR alpha and the resulting feeling of satiety is a new element in the complex and high evolved steering and regulation of food intake.

In another special form of the invention, the said substance is chemically modified to increase its hydrophibility which will make it more bio-available.

In a further form of the invention, the CLA is offered in a mixture with other lipophilic substances, preferably other unsaturated fatty acids which are also subject to the invention.

In a further form of the invention, said CLA, is offered in a mixture with other lipophilic substances, preferably medium-chain triglycerides, which will be even more easily quantitatively transported to the liver, and therefore the effective concentration of free fatty acids to induce the synthesis of keton bodies will be reached even faster, in a synergistic manner.

### Form of application

The CLA is usually applied orally ―in form of a functional food and dietary supplement agent as "food product" or as medicament with a physiological acceptable carrier in form of, but not limited to, tablets, capsules, pills, pellets, granules, powders, emulsions, suspensions or syrups.
"Physiologically acceptable carrier" refers to any carrier or excipient commonly used with oily pharmaceuticals. Such carriers or excipients include, but are not limited to, inert diluents such as calcium carbonate, sodium carbonate, lactose, sucrose, sorbitol, mannitol, calcium phosphate or sodium phosphate; granulating and disintegrating agents, such as starch and alginic acid; binding agents such as starch, gelatin or acacia; and lubricating agents such as magnesium stearate, stearic acid or talc. Tablets may be uncoated or may be coated with known techniques to control delivery and absorption.
Formulations for oral use may also be administered as hard gelatin capsules wherein the active ingredient is mixed with an inert solid diluent, for example calcium carbonate, calcium phosphate or kaolin, lactose, mannitol or as soft gelatin capsules wherein the active ingredient is mixed with water or an oil medium, such as peanut oil, liquid paraffin, middle chain triglycerides or olive oil.
As used herein, the term "food product" refers to any food or feed suitable for consumption by humans, non-ruminant animals, or ruminant animals. The "food product" may be a prepared and packaged food or an animal feed (e.g. , extruded and pelleted animal feed or coarse mixed feed).

## Claims

1. Use of the *cis-9,trans-*11 isomer of conjugated linoleic acid (CLA) for the manufacture of a dietary supplement, a functional food, or a medicament for reducing appetite or food intake.

2. The use according to claim 1, wherein conjugated linoleic acid (CLA) is used, comprising the *cis-*9,*trans*-11 isomer in amounts of at least 30 % b.w. - calculated on the total CLA content - for the manufacture of a dietary supplement, a functional food, or a medicament for reducing appetite or food intake.

3. The use according to claim 1 or 2, wherein conjugated linoleic acid (CLA) is used, comprising at most 30 % b.w. *trans*-10,*cis*-12 isomers and in total less than 1 % b.w. 8,10-, 11,13 and *trans,trans* isomers - calculated on the total CLA conten t- for the manufacture of a dietary supplement, a functional food, or a medicament for reducing appetite or food intake.

4. The use according to any of claims 1 to 3, wherein conjugated linoleic acid (CLA) is used, comprising as main isomers the *cis*-9,*trans*-11 isomer and the *trans*-10, *cis*-12 isomer in a ratio 1 : 1.2 to 1.2 to 1 for the manufacture of a dietary supplement, a functional food, or a medicament for reducing appetite or food intake.

5. Use according to any one of claims 1 to 4, **characterised in that** said substance is applied in amounts of from 0.05 to 5 g/day.

6. Use according to any of claims 1 to 4, **characterised in that** conjugated linoleic acid (CLA) is combined with other lipophilic substances.

## Patentansprüche

1. Verwendung des *cis*-9,*trans*-11-Isomers von konjugierter Linolsäure (CLA) zur Herstellung eines Nahrungsergänzungsmittels, eines funktionellen Nahrungsmittels oder eines Medikaments zum Verringern des Appetits oder der Nahrungsaufnahme.

2. Verwendung gemäß Anspruch 1, wobei konjugierte Linolsäure (CLA), welche das *cis*-9, *trans*-11-Isomer in Mengen von wenigstens 30 Gew.-%, bezogen auf den gesamten CLA-Gehalt, umfasst, zur Herstellung eines Nahrungsergänzungsmittels, eines funktionellen Nahrungsmittels oder eines Medikaments zum Verringern des Appetits oder der Nahrungsaufnahme verwendet wird.

3. Verwendung gemäß Anspruch 1 oder 2, wobei konjugierte Linolsäure (CLA), welche höchstens 30 Gew.-% *trans*-10,*cis*-12-Isomere und insgesamt weniger als 1 Gew.-% 8,10-, 11,13- und *trans,trans*-Isomere, bezogen auf den gesamten CLA-Gehalt, umfasst, zur Herstellung eines Nahrungsergänzungsmittels, eines funktionellen Nahrungsmittels oder eines Medikaments zum Verringern des Appetits oder der Nahrungsaufnahme verwendet wird.

4. Verwendung gemäß einem der Ansprüche 1 bis 3, wobei konjugierte Linolsäure (CLA), welche als Hauptisomere das *cis*-9,*trans*-11-Isomer und das *trans-*10,*cis*-12-Isomer in einem Verhältnis von 1 : 1,2 bis 1,2 : 1 umfasst, zur Herstellung eines Nahrungsergänzungsmittels, eines funktionellen Nahrungsmittels oder eines Medikaments zum Verringern des Appetits oder der Nahrungsaufnahme verwendet wird.

5. Verwendung gemäß einem der Ansprüche 1 bis 4, **dadurch gekennzeichnet, dass** der Stoff in Mengen von 0,05 bis 5 g/Tag zur Anwendung kommt.

6. Verwendung gemäß einem der Ansprüche 1 bis 4, **dadurch gekennzeichnet, dass** konjugierte Linolsäure (CLA) mit anderen lipophilen Stoffen kombiniert wird.

## Revendications

1. Utilisation de l'isomère cis-9,trans-11 de l'acide linoléique conjugué (ALC) pour la préparation d'un complément alimentaire, d'un aliment fonctionnel ou d'un médicament pour diminuer l'appétit ou l'apport alimentaire.

2. Utilisation selon la revendication 1, selon laquelle on utilise l'acide linoléique conjugué (ALC), comprenant l'isomère cis-9,trans-11 en quantité d'au moins 30 % en poids, calculées sur la teneur totale en ALC, pour la préparation d'un complément alimentaire, d'un aliment fonctionnel ou d'un médicament pour diminuer l'appétit ou l'apport alimentaire.

3. Utilisation selon la revendication 1 ou 2, selon laquelle on utilise de l'acide linoléique conjugué (ALC), comprenant au plus 30 % en poids d'isomères trans-10,cis-12 et au total moins de 1 % en poids d'isomères 8,10-,11,13 et trans,trans, calculés sur la teneur totale en ALC, pour la préparation d'un complément alimentaire, d'un aliment fonctionnel ou d'un médicament pour diminuer l'appétit ou l'apport alimentaire.

4. Utilisation selon l'une quelconque des revendications 1 à 3, selon laquelle on utilise de l'acide linoléique conjugué (ALC), comprenant comme isomères principaux l'isomère cis-9,trans-11 et l'isomère trans-10,cis-12 selon un rapport de 1/1,2 à 1,2/1 pour la préparation d'un complément alimentaire, d'un aliment fonctionnel ou d'un médicament pour diminuer l'appétit ou l'apport alimentaire.

5. Utilisation selon l'une des revendications 1 à 4,
**caractérisée en ce que**
ladite substance est appliquée en quantités allant de 0,05 g à 5 g/jour.

6. Utilisation selon l'une des revendications 1 à 4,
**caractérisée en ce que**
l'acide linoléique conjugué (ALC) est combiné à d'autres substances lipophiles.
